Europäisches Patentamt

European Patent Office

Office européen des brevets

(19)

(11) Numéro de publication: **0 159 248 B1**

(12)

# FASCICULE DE BREVET EUROPEEN

(45) Date de publication du fascicule du brevet:
08.11.89

(51) Int. Cl.⁴: **A 61 B 6/03**

(21) Numéro de dépôt: **85400589.9**

(22) Date de dépôt: **26.03.85**

(54) **Procédé de reconstruction d'une image à haute résolution, par tomodensitométrie.**

(30) Priorité: **30.03.84 FR 8405042**

(43) Date de publication de la demande:
**23.10.85 Bulletin 85/43**

(45) Mention de la délivrance du brevet:
**08.11.89 Bulletin 89/45**

(84) Etats contractants désignés:
**DE GB IT NL**

(56) Documents cités:
**DE-A- 2 655 000**
**FR-A- 2 227 582**
**FR-A- 2 391 696**
**GB-A- 1 474 685**
**GB-A- 1 577 046**

(73) Titulaire: **GENERAL ELECTRIC CGR S.A., 13, Square Max-Hymans, F-75015 Paris (FR)**

(72) Inventeur: **Tan, Siv-Cheng, THOMSON-CSF SCPI 173, bld Haussmann, F-75379 Paris Cedex 08 (FR)**
Inventeur: **Le Gall, Didier, THOMSON-CSF SCPI 173, bld Haussmann, F-75379 Paris Cedex 08 (FR)**
Inventeur: **Benchimol, Claude, THOMSON-CSF SCPI 173, bld Haussmann, F-75379 Paris Cedex 08 (FR)**

(74) Mandataire: **Ballot, Paul Denis Jacques et al, Cabinet Ballot-Schmit 84, avenue Kléber, F-75116 Paris (FR)**

ACTORUM AG

## Description

L'invention concerne un procédé de reconstruction d'une image à haute résolution, par tomodensitométrie, ayant pour but d'améliorer la qualité de l'image reconstruite sans alourdir la structure technologique des moyens de mesure de l'absorption de rayonnement pénétrant et sans modifier sensiblement le processus de prise des données.

Les tomodensitomètres dits de «troisième génération» se composent essentiellement d'une source de rayonnement pénétrant (des rayons X) émettant un faisceau plat en éventail, d'une rangée courbe de détecteurs placés en regard de cette source pour mesurer la fraction non absorbée du rayonnement dans le plan de la coupe à imager, de moyens pour faire tourner la source et les détecteurs dans le plan de cette coupe et d'un ordinateur pour traiter les données fournies par les détecteurs et reconstruire une image à partir de ces données. La résolution spatiale de l'image reconstruite dépend notamment du nombre des détecteurs embrassant l'éventail du faisceau de rayons X. Pour une ouverture de faisceau donnée, plus le nombre de détecteurs est élevé et plus la résolution spatiale est bonne. Cependant, l'ensemble multi-détecteurs est l'un des organes les plus coûteux du tomodensitomètre et l'un de ceux qui est le plus difficile à fabriquer avec une bonne fiabilité. Par conséquent l'augmentation du nombre de détecteurs sur un secteur donné correspondant à l'éventail, se heurte à des limites technologiques.

L'un des buts de l'invention est d'améliorer sensiblement la qualité de l'image sans modifier la structure technologique de l'ensemble source-détecteurs et sans modifier sensiblement la procédure de prise des données proprement dite.

Un autre but de l'invention est aussi de réduire le coût de construction d'un tomodensitomètre ayant une résolution spatiale comparable à celle des tomodensitomètres existants, cette réduction de coût étant obtenue en utilisant un ensemble multi-détecteurs comportant deux fois moins de détecteurs que les machines existantes. Une solution à ce problème est indiquée dans la demande de brevet français FR-A-2 391 696. Cependant la mise en œuvre de cette solution nécessite des calculs complexes.

Dans cet esprit, l'invention concerne essentiellement un procédé de reconstruction d'images par tomodensitométrie, du type consistant à faire tourner un ensemble source-détecteurs dans le plan d'une coupe à imager, ladite source émettant un faisceau de rayonnement pénétrant en forme d'éventail et à relever et mémoriser une succession de vues pour différents angles $\beta$ de rotation de l'ensemble source-détecteurs par rapport à un système d'axes de référence fixe dudit plan, les détecteurs étant agencés pour converger vers le foyer de ladite source et chaque vue étant échantillonnée pour des angles $\gamma$ représentatifs des détecteurs et repérés à partir de la droite joignant le centre de rotation de l'ensemble source-détecteurs et ledit foyer, le décalage angulaire entre deux détecteurs adjacents étant $\Delta\gamma$ constant et le décalage angulaire $\Delta\beta$ entre vues

étant constant, le décalage angulaire entre ladite droite et le détecteur le plus proche de celle-ci étant réglé à $\left[\dfrac{\Delta\gamma}{4}\right]$,

– à relever et mémoriser une série de vues réparties sur un tour complet dudit ensemble source-détecteurs;

– à élaborer pour chaque vue des valeurs intermédiaires représentant un échantillonnage entrelacé avec celui desdits détecteurs, chaque valeur intermédiaire correspondant à un angle $\gamma$ d'échantillonnage donné étant obtenue par une combinaison d'au moins deux valeurs d'absorption linéaires, caractérisé en ce que ces valeurs sont déduites de valeurs de mesure prélevées dans des vues consécutives situées de part et d'autre d'une vue fictive dont l'image par la transformation $T(\theta,\gamma) = (\theta + [2k-1]\pi, -\gamma)$ contient le point représentatif de ladite valeur intermédiaire dans l'espace $[\theta,\gamma]$ avec $\theta = \beta + \gamma$ et $k$ entier relatif, lesdites valeurs de mesure desdites vues étant prélevées sur le détecteur d'angle opposé à celui de ladite valeur intermédiaire recherchée; et

– à appliquer un algorithme de reconstruction, connu en soi, comprenant une convolution et une rétroprojection aux vues ainsi complétées par lesdites valeurs intermédiaires.

L'invention sera mieux comprise à la lumière de la description qui va suivre d'un procédé conforme à son principe et des éléments constitutifs essentiels d'un tomodensitomètre fonctionnant selon ce procédé, faite en référence aux dessins annexés dans lesquels:

la figure 1 est une représentation schématique des principaux éléments constitutifs d'un tomodensitomètre mettant en œuvre l'invention;

la figure 2 est une représentation dans l'espace $[\theta,\gamma]$ des vues prises par un tomodensitomètre classique fonctionnant en mode pulsé pour la reconstruction d'une image;

la figure 3 est une représentation dans le même espace $[\theta,\gamma]$ des vues prises par un tomodensitomètre du même type dans lequel on a effectué l'une des modifications nécessaires à la mise en œuvre de l'invention, à savoir un décalage statique entre la source et les détecteurs;

la figure 4 illustre, dans l'espace $[\theta,\gamma]$ une transformation applicable aux vues de la figure 3 pour une mise en œuvre du procédé de l'invention;

la figure 5 illustre dans l'espace $[\theta,\gamma]$ le résultat de cette transformation et montre le processus d'interpolation permettant de compléter les vues;

la figure 6 illustre la mise en œuvre d'un algorithme conforme à l'invention, pour le calcul d'une valeur intermédiaire de la première vue $V_1$;

la figure 7 illustre une phase de calcul du même algorithme; et

la figure 8 est une représentation dans l'espace $[\theta,\gamma]$ d'une vue, dans le cas d'un tomodensitomètre fonctionnant en mode continu.

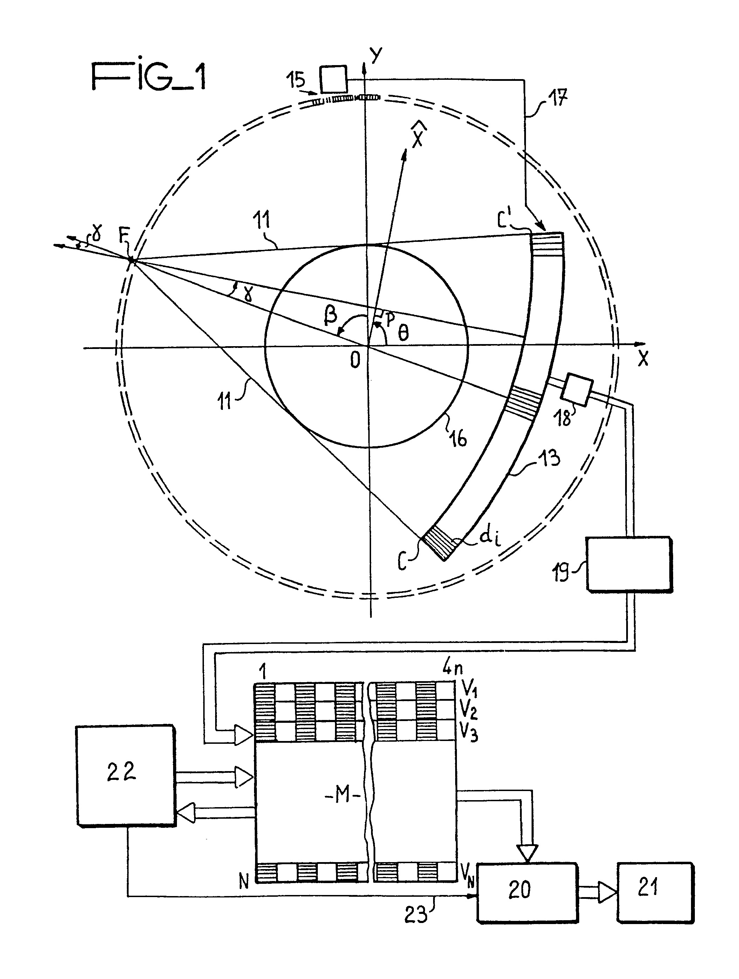

La suite de la description en référence aux figures 1 à 7, se place dans le cas particulier d'un tomodensitomètre fonctionnant en mode pulsé. Ce genre de tomodensitomètre est bien connu. Dans la plupart des cas, le mode pulsé se concrétise par le fait que le faisceau en éventail n'est émis que pendant un intervalle de temps très bref au moment où l'ensemble source-détecteurs se trouve à un angle β prédéterminé. Dans ce cas, il suffit que la lecture de tous les détecteurs soit achevée lorsque l'ensemble source-détecteurs parvient à l'angle β suivant. Cependant, une autre façon de réaliser ledit mode pulsé peut consister à générer en permanence le faisceau en éventail mais à «lire» tous les détecteurs simultanément à chaque angle β.

En se reportant à la figure 1, on a représenté les éléments constitutifs essentiels d'un tomodensitomètre. Cet appareil comporte un ensemble source-détecteurs mobile en rotation dans un plan de coupe à imager. Le centre de rotation est O et le plan de la coupe sera considéré comme étant celui de la figure 1. On associe à ce plan de coupe un système d'axes de référence fixe XOY, orthonormés. La source est symbolisée sur le dessin par son foyer F. De façon bien connue, il s'agit d'une source de rayonnement pénétrant et plus particulièrement des rayons X, émettant un faisceau 11 en forme d'éventail. Dans la suite du texte, on parlera simplement de la source F. Le boîtier 13 renfermant tous les détecteurs $d_i$ a une forme courbe pour que ces détecteurs soient agencés suivant un arc de cercle $\overset{\frown}{CC'}$ géométriquement centré sur le foyer F. On sait que le boîtier peut aussi être rectiligne mais renfermer des détecteurs agencés pour converger vers le foyer. Dans l'exemple décrit, le faisceau en éventail 11 embrasse l'arc de cercle $\overset{\frown}{CC'}$ occupé par les faces d'entrée des détecteurs $d_i$. Ainsi, l'image reconstituée sera à l'intérieur du cercle 16 de centre O, inscrit dans les limites de l'angle $\overset{\wedge}{CFC'}$. On peut utiliser un ensemble comportant 1024 détecteurs $d_i$. Le boîtier 13 et la source F sont solidaires d'un même support tournant (centre de rotation O) et fixes l'un par rapport à l'autre de sorte qu'on peut repérer la rotation de l'ensemble source-détecteurs par l'angle β entre l'angle OY et la droite OF. Les valeurs d'atténuation du faisceau de rayons X pour des angles β prédéterminés sont déduites des valeurs de mesure prélevées aux sorties des détecteurs et mémorisées dans une mémoire M d'un ordinateur. Pour chaque angle β prédéterminé, un ordre de lecture des détecteurs $d_i$ est émis, par exemple au moyen d'un codeur incrémental 15 agencé pour lire des informations d'angle solidaires de l'ensemble tournant source-détecteurs et pour délivrer (liaison de pilotage 17) des ordres de commande de lecture de l'état de tous les détecteurs. L'ensemble des valeurs d'atténuation linéaire déduites des lectures des détecteurs pour un angle β donné est appelée «vue».

On peut donc considérer que chaque vue est échantillonné de par la structure même de l'ensemble de détecteurs, pour des angles γ représentatifs des détecteurs. Chaque angle γ indiquant la position d'un détecteur $d_i$ est repéré à partir de la droite OF. De ce fait, on appelera Δγ le décalage angulaire constant entre les centres des faces d'entrée de deux détecteurs adjacents.

Si on considère pour chaque angle β d'une vue, l'axe $O\overset{\wedge}{X}$ perpendiculaire en P au rayon d'angle γ frappant un détecteur quelconque, on démontre facilement que l'angle θ entre l'axe OX du repère fixe et l'axe $O\overset{\wedge}{X}$ est tel que:

$$\theta = \beta + \gamma$$

ceci dans tous les cas de figures, les angles θ, β et γ étant orientés. On a encore:

$$\gamma = \theta - \beta$$

D'après cette dernière relation, on voit que les différentes vues (pour des angles β différents) ont une représentation extrêmement simple dans l'espace [θ, γ].

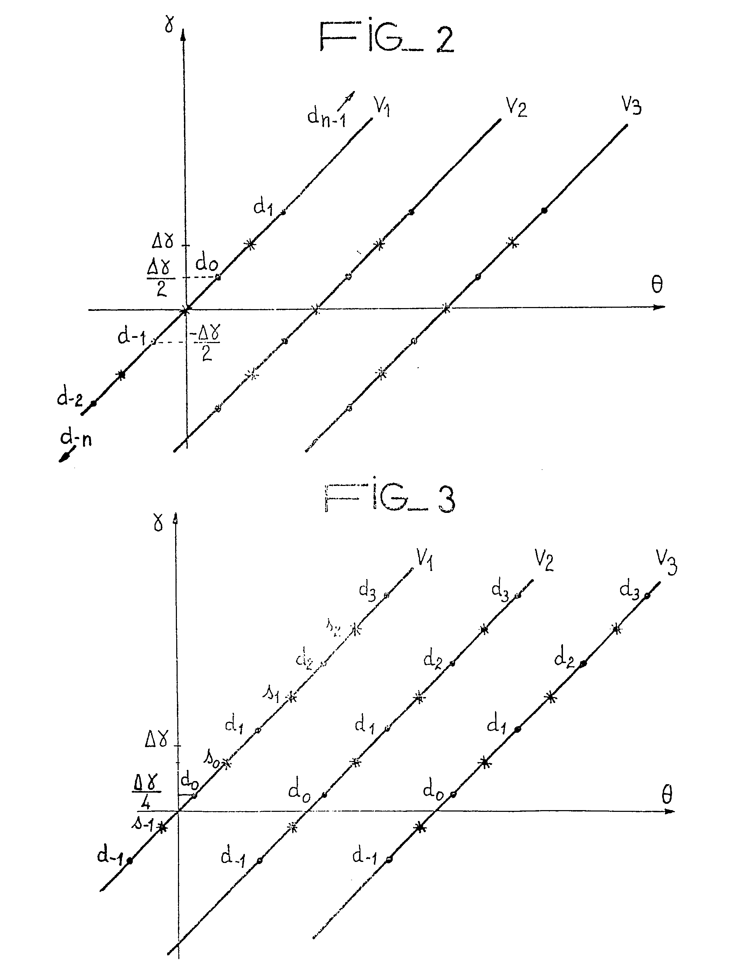

Comme le montre la figure 2, ces vues sont portées par des droites parallèles à la première bissectrice et décalées de Δβ le long de l'axe des $\overrightarrow{\theta}$. On peut porter sur ces droites des points représentant les coordonnées, dans l'espace [θ, γ] des valeurs échantillonnées effectivement acquises à la lecture des différentes vues.

Ainsi, si on suppose que le nombre de détecteurs est pair, soit 2n et que la droite OF passe exactement entre les deux détecteurs du centre $d_{-1}$ et $d_0$, ceux-ci apparaissent décalés de $\left[\dfrac{\Delta\gamma}{2}\right]$ de part et d'autre de l'axe $\overrightarrow{\gamma}$. Le but de l'invention est d'obtenir pour chaque vue des valeurs échantillonnées intermédiaires dont les coordonnées dans l'espace [θ,γ] sont figurées par des étoiles * sur la figure 2. Autrement dit, le but de l'invention est d'obtenir des vues comportant 4n valeurs échantillonées avec un ensemble de 2n détecteurs seulement et d'améliorer ainsi la résolution spatiale de l'image reconstruite.

On provoque un décalage angulaire permanent et prédéterminé entre l'ensemble des détecteurs (c'est-à-dire le boîtier 13) et la droite OF. Le décalage angulaire entre la droite OF et le détecteur le plus proche ($d_0$) est choisi égal à $+ \dfrac{\Delta\gamma}{4}$. Cela signifie que l'ensemble des détecteurs est décalé «à gauche» en considérant la figure 1, de façon que le centre de la face d'entrée du détecteur $d_0$ soit décalé angulairement de $+ \dfrac{\Delta\gamma}{4}$ par rapport à OF et que le détecteur $d_{-1}$ soit décalé de $- \dfrac{3\Delta\gamma}{4}$ par rapport à cette même droite.

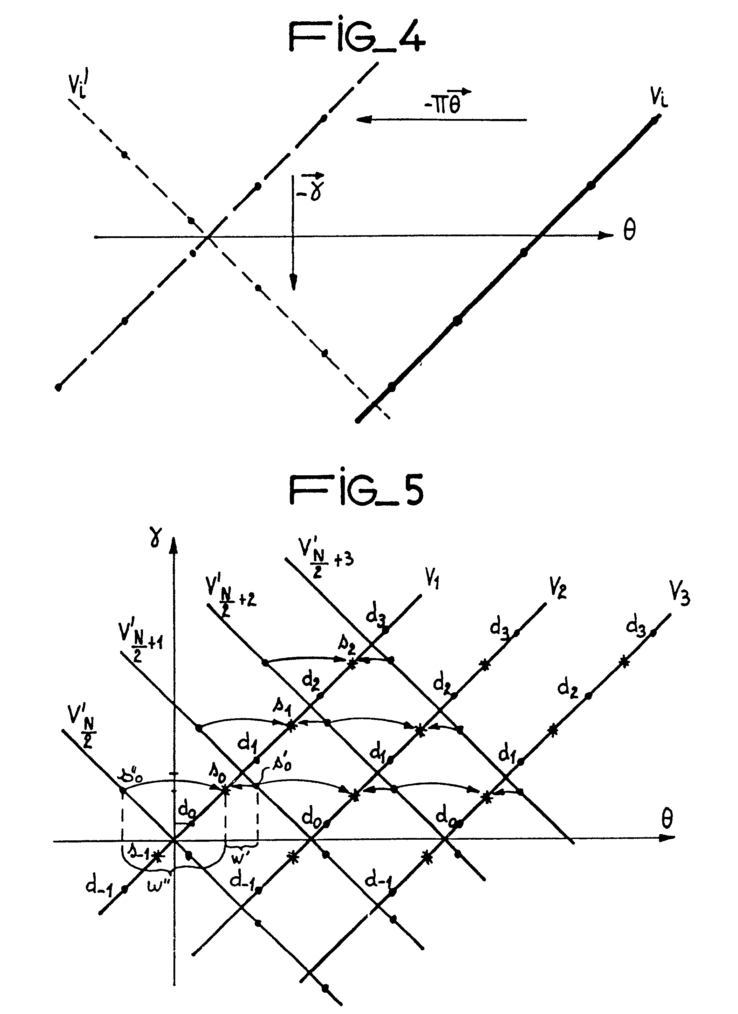

La figure 3 montre la configuration des vues dans l'espace [θ,γ] après ce décalage.

Pour la mise en œuvre de l'invention, on relève une série $V_i$ de N vues réparties sur un tour complet de l'ensemble source-détecteurs, avec $N = \dfrac{2\pi}{\Delta\beta}$.

Les valeurs intermédiaires recherchées $s_i$ sont également repérées sur les différentes vues de la figure 3, chaque emplacement de valeur intermédiaire dans l'espace [θ,γ] étant équidistant des emplacements de deux détecteurs réels voisins.

Soit la transformation:

$$T(\theta,\gamma) = (\theta - \pi, -\gamma)$$

On peut démontrer que la fonction d'absorption d'une vue est invariante par cette transformation T. Or cette transformation T peut se décomposer dans

l'espace $[\theta, \gamma]$ en une translation $- \overrightarrow{\pi\theta}$ suivie d'une symétrie par rapport à $\overrightarrow{\theta}$. Cette transformation est illustrée en soi à la figure 4. Si on applique ladite transformation à la vue $V_i$, le résultat est la «vue» $V_i'$.

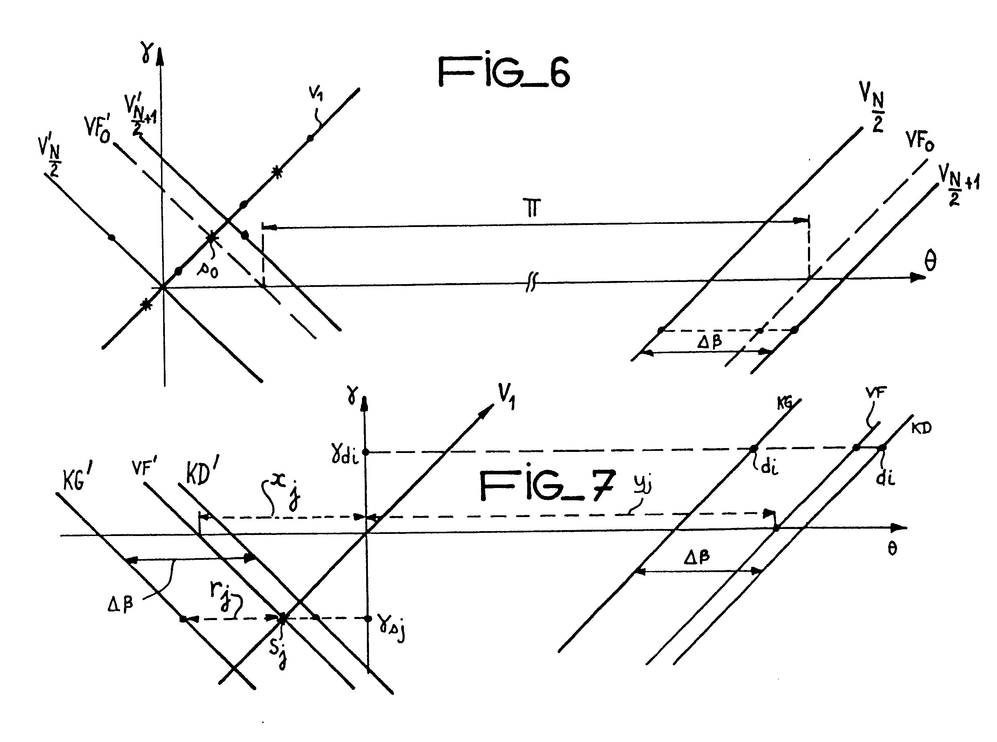

Si on applique ainsi la transformation T à la série des vues $V_i$, on constate que les valeurs échantillonnées des «vues» $V_i'$ transformées ont la même ordonnée $\gamma$ que des valeurs intermédiaires $s_i$ recherchées. On peut donc compléter les vues $V_i$ par des interpolations à partir de valeurs d'absorption linéaires correspondant à des valeurs échantillonnées des «vues» $V_i'$, à $\gamma$ constant, dans l'espace $[\theta, \gamma]$, comme indiqué par les flèches sur la figure 5. Selon l'exemple, chaque valeur intermédiaire d'angle $\gamma$ donné est obtenue par une combinaison de deux valeurs d'absorption linéaire déduites des valeurs échantillonnées de même ordonnée $\gamma$, prélevées dans des «vues» $V_i'$ situées de part et d'autre de la valeur intermédiaire recherchée. La combinaison peut simplement consister en une interpolation linéaire entre les valeurs d'absorption linéaire correspondantes de deux «vues» adjacentes $V_i'$, comme indiqué figure 5. Autrement dit, chaque valeur intermédiaire est obtenue en faisant la moyenne entre ces deux valeurs d'absorption linéaire précitées, $s_0'$ et $s_0''$, en les pondérant par l'inverse $(1/w'$ et $1/w'')$ de l'écart (en projection sur l'axe des $\overrightarrow{\theta}$, figure 5) entre leur position respective, $s_0'$ et $s_0''$, et la position, $s_0$, de la valeur intermédiaire recherchée. Pour certaines «vues» $V_i'$ nécessaires aux calculs d'interpolation suggérés par la figure 5, la transformation T fait appel à une vue $V_i$ située en dehors de l'intervalle $[\theta, 2\pi]$ des vues réellement acquises. Cependant, ces dernières peuvent être théoriquement prolongées dans l'espace $[\theta, \gamma]$ de $-\infty$ à $+\infty$ en raison de la périodicité par rapport à $\theta$, de la fonction d'atténuation linéaire au-delà d'un tour complet (en raison de la condition $N = \dfrac{2\pi}{\Delta\beta}$), ce qui peut se traduire ainsi:

$$V_i = V_j \leftrightarrow i = j \text{ Modulo } N$$

Autrement dit, la transformation T peut être complétée de la façon suivante:

$$T(\theta, \gamma) = (\theta + (2k - 1)\pi, -\gamma)$$

avec k entier relatif.

Il existe toujours une valeur de k telle que la vue $V_i$ sur laquelle est appliquée la translation $\theta + (2k - 1)\pi$ soit bien l'une des vues acquises $V_i$. La figure 5 illustre la justification du processus de l'invention. Ce mode de calcul des valeurs intermédiaires doit maintenant être explicité car les «vues» $V_i'$ ne représentent qu'un réarrangement des vues $V_i$ et le calcul de chaque valeur intermédiaire peut se faire en fait à partir de chaque «emplacement» de valeur intermédiaire recherchée, dans l'espace $[\theta, \gamma]$. C'est ce qu'illustre la figure 6 où on n'a représenté que la première vue $V_1$, ainsi que deux «vues» $V_i'$, résultat de la transformation T appliquée aux vues $V_{N/2}$ et $V_{N/2} + 1$ (N étant pair), lesquelles sont aussi représentées. Les Vues $V_{N/2}$ et $V_{N/2} + 1$ sont situées de part et d'autre d'une vue fictive $VF_0$ dont la transformée $VF_0'$ par la transformation T, passe par les coordonnées de la valeur intermédiaire $s_0$ de la vue $V_1$ dans l'espace $[\theta, \gamma]$. Selon l'invention, il suffit donc pour chaque valeur intermédiaire $(s_0)$ de chaque vue $(V_1)$, de rechercher la «vue fictive» $(VF_0)$ correspondante dont la transformée par la transformation T passe par les coordonnées de ladite valeur intermédiaire, de sélectionner les deux vues réelles adjacentes $V_i$, $V_{i+1}$ $(V_{N/2}, V_{N/2} + 1)$ situées de part et d'autre de ladite vue fictive, de prélever dans ces deux vues les valeurs d'absorption d'angle $-\gamma_{\Delta j}$ $(\gamma s_0$ étant l'ordonnée de $s_0$ dans l'espace $[\theta, \gamma])$ qui correspondent à des valeurs de mesure d'un même détecteur réel, et d'effectuer une interpolation linéaire entre ces valeurs d'absorption linéaire. Il est à noter que, pour simplifier, on a supposé ci-dessus que le nombre N de vues acquises $V_i$ était pair; le processus de l'invention s'applique néanmoins tel quel si N est impair.

On va maintenant décrire l'un des algorithmes de calcul possibles, constituant une mise en oeuvre actuellement préférée du procédé décrit ci-dessus. Dans l'exemple décrit, on se place dans le cas d'un décalage des détecteurs «à gauche» tel qu'illustré sur les dessins et dans le cas d'un mode de fonctionnement pulsé où, comme on sait, les valeurs échantillonnées des vues $V_i$ sont portées par des droites parallèles à la première bissectrice de l'espace $[\theta, \gamma]$. On va d'abord décrire en référence à la figure 7, l'algorithme permettant de compléter la vue $V_1$. L'équation de la vue $V_1$ est:

$$\theta = \gamma$$

On cherche à calculer la valeur intermédiaire en $s_j$. Soit $x_j$ l'écart entre l'intersection de la vue à compléter avec l'axe $\overrightarrow{\theta}$ (en l'occurence l'origine, dans le cas de la vue $V_1$) et l'interconnection de la transformée de la vue fictive (passant par $s_j$) avec le même axe $\overrightarrow{\theta}$.

On a: $\quad\quad\quad x_j = 2\gamma s_j \quad\quad\quad\quad\quad (A)$

Le point d'intersection de la vue fictive correspondante avec l'axe des $\overrightarrow{\theta}$ est donc:

$$y_j = \pi + x_j \quad\quad\quad\quad\quad (B)$$

Le numéro de la vue réelle KG situé «à gauche» de cette vue fictive est donc déterminé par la valeur entière du rapport $\dfrac{y_j}{\Delta\beta}$.

De façon plus précise:

$$KG_{(j)} = \left[\left(\frac{y_j}{\Delta\beta}\right)\right] + 1, \text{ modulo } N \quad\quad (C)$$

et le numéro de la vue KD située «à droite» de la vue fictive est donc:

$$KD_{(j)} = KG_{(j)} + 1, \text{modulo } N \quad\quad (D)$$

Il reste maintenant à déterminer «l'ordonnée» $\gamma \, d_i$ d'un détecteur $d_i$ à prendre en compte dans chaque vue KG et KD, soit, d'après la transformation T:

$$-\gamma d_i = \gamma s_j$$

Or si on se reporte à la figure 3:

$$\gamma d_i = \frac{\Delta\gamma}{4} + i\,\Delta\gamma$$

$$\gamma s_j = -\frac{\Delta\gamma}{4} + (j+1)\,\Delta\gamma$$

soit:　　　　$i = -j - 1$　　　　　　(E)

D'autre part, soit $r_j$ le reste de l'opération $\left[ E\left(\dfrac{\gamma_j}{\Delta\beta}\right)\right]$, $r_j$ représente l'écart en projection sur l'axe des $\overrightarrow{\theta}$ entre la position de $s_j$ et celle du détecteur $d_i$ dans la vue réelle KG; $r_j$ est représenté sur la figure 7 entre la transformée de la vue fictive, passant par $s_j$ et la transformée KG' de KG. Par conséquent, on peut déduire de $r_j$ la pondération à apporter aux valeurs d'atténuation linéaire correspondant aux valeurs de mesure prélevées sur le détecteur $d_i$ des vues KG et KD.

Soient $h_{KD}(d_i)$ et $h_{KG}(d_i)$, les valeurs d'atténuation linéaire correspondant au détecteur $d_i$ dans les vues réelles $KD_{(j)}$ et $KG_{(j)}$, et soit:

$$R_{(j)} = \frac{r_j}{\Delta\beta} \; ;$$

on vérifie que la valeur d'atténuation linéaire $h(s_j)$ à attribuer à l'emplacement $s_j$ de la vue $V_1$ (autrement dit la valeur intermédiaire $s_j$ elle-même) s'exprime:

$$h(s_j) = R_{(j)}\,[h_{KD}(d_i) - h_{KG}(d_i)] + h_{KG}(d_i)　　　　(F)$$

La programmation des expressions A à F ci-dessus est à la portée de l'homme du métier tout comme la conception d'un processeur de calcul spécialisé couplé à la mémoire tampon M.

Pour compléter les vues suivantes $V_m$ (c'est-à-dire $V_2, V_3, ..., V_N$) on a, au lieu de la relation (A):

$$x_j - (m-1)\Delta\beta = 2\gamma s_j$$

soit:
$$x_j = 2\gamma s_j + (m-1)\,\Delta\beta　　　　(A')$$

L'expression A' remplace A dans l'algorithme de réarrangement et d'interpolation mais les autres relations B à F restent inchangées. Il est aussi intéressant de remarquer que d'une vue à l'autre $R_{(j)}$ reste inchangé et que $KG_{(j)}$ est incrémenté de 1. Par conséquent, il est possible dans le logiciel de calcul, de précalculer r et KG uniquement pour la première vue et de procéder ensuite par incrémentations de KG. Par ailleurs, on vérifie que le même algorithme est valable pour un décalage des détecteurs «à droite», de sorte que le procédé décrit s'applique pour un décalage de l'ensemble de détecteurs de : $\left|\dfrac{\Delta\gamma}{4}\right|$.

L'algorithme qui précède a été développé avec l'hypothèse d'un fonctionnement en mode pulsé. Il est facile de l'adapter à un fonctionnement en mode continu. Selon un mode de fonctionnement continu usuel, on suppose que la source S émet en permanence et que la lecture des valeurs de mesure aux sorties des détecteurs, pour chaque vue, se fait par scrutation progressive d'une extrémité à l'autre de l'ensemble des détecteurs, par exemple depuis le détecteur $d_{-n}$ jusqu'au détecteur $d_{n-1}$.

Deux paramètres supplémentaires doivent donc être pris en considération:
— la vitesse de rotation, v, de l'ensemble source-détecteurs;
— l'intervalle de temps $\tau$ séparant l'acquisition des valeurs échantillonnées correspondant à deux détecteurs voisins, en considérant une vitesse de scrutation constante.

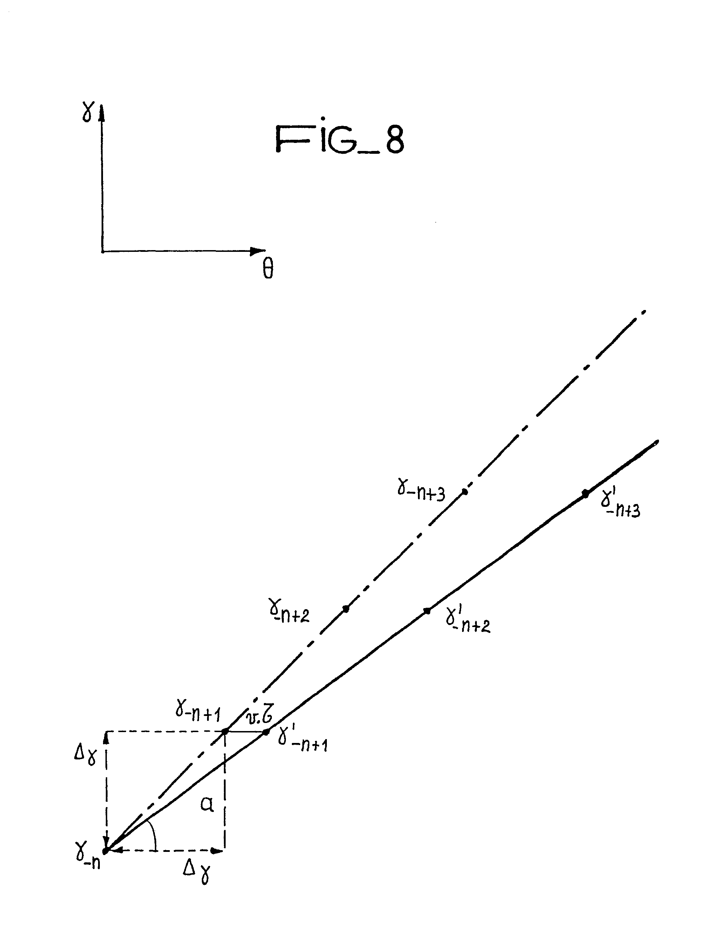

La figure 8 illustre la façon dont la représentation d'une vue dans l'espace $[\theta, \gamma]$ évolue si on prend en compte les paramètres v et $\tau$. La vue portée par la droite en trait interrompu correspond au mode pulsé tandis que la même vue portée par la droite en trait plein correspond au mode continu.

Les valeurs échantillonnées se déplacent parallèlement à l'axe $\overrightarrow{\theta}$ puisque $\theta = \beta + \gamma$; $\beta$ variant pendant l'acquisition. Les deux droites concourrent en $d_{-n}$ puisque la scrutation des détecteurs commence par ce détecteur et la valeur échantillonnée suivante, $d_{-n+1}$ se trouve déplacée en $d'_{-n+1}$ d'une distance v. $\tau$ dans l'espace $[\theta, \gamma]$, parallèlement à l'axe des $\overrightarrow{\theta}$.

Soit $â$ l'angle entre $\overrightarrow{d_{-n}d_{-n+1}}$ et $\overrightarrow{d_{-n}d'_{-n+1}}$.

$$tga = \frac{\Delta\gamma}{\Delta\gamma + v.\tau} = \frac{1}{1 + \dfrac{v.\tau}{\Delta\gamma}}$$

soit　　$\alpha = \dfrac{v.\tau}{\Delta\gamma}$,　　$tga = \dfrac{1}{1 + \alpha}$

Les vues sont donc portées par des droites parallèles à la droite d'équation $\theta = (1 + \alpha)\,\gamma$.

Par conséquent, pour la vue $V_1$ la relation (A) devient:

$$x_j = 2\,(1 + \alpha)\,\gamma\,s_j$$

et pour une vue $V_m$ quelconque, la relation (A') devient:

$$x_j = 2\,(1 + \alpha)\,\gamma\,s_j + (m-1)\,\Delta\beta$$

Le reste de l'algorithme s'applique sans modification avec les nouvelles valeurs de $x_j$ définies ci-dessus.

On a vérifié que le procédé décrit s'applique aussi sans changement quelque soit le type de scrutation en mode continu. Dans ce cas, les valeurs échantillonnées des vues ne sont plus obligatoirement portées par des droites mais par des courbes quelconques auxquelles on peut appliquer la même transformation T pour en déduire les valeurs intermédiaires recherchées à partir d'interpolations analogues à celles décrites plus haut.

En se reportant à nouveau à la figure 1, on a décrit sous forme de schéma-bloc fonctionnel les parties essentielles de l'ordinateur associé aux détecteurs $d_i$. Les valeurs échantillonnées lues aux sorties des détecteurs $d_i$ sont transformées en informations numériques grâce à un convertisseur analogique-numérique 18 puis traitées dans un préprocesseur 19 effectuant les opérations habituelles de calibration et de transformation logarithmique. Les informations ainsi transformées sont les valeurs d'atténuation linéaires correspondantes et ces informations numériques sont mémorisées dans la mémoire M.

Pour des raisons qui apparaîtront clairement plus loin, la mémoire M se présente sous forme d'une matrice d'unités de mémorisation comprenant 4 n colonnes et N lignes (nombre de vues $V_i$) puisque l'acquisition des vues $V_i$ se fait sur un tour complet et avec: $\Delta\beta = \dfrac{2\pi}{N}$.

Le remplissage de la mémoire M par les informations représentatives des vues $V_i$ se fait à raison d'une unité de mémorisation sur deux; ces unités sont représentées en grisé sur la figure 1. Le remplissage de la mémoire M tel que représenté, correspond à un décalage «à gauche» des détecteurs. La mémoire M est associée à des moyens de lecture 20, qui organisent le transfert des informations vers un processeur 21, connu en soi, et dont le rôle est d'appliquer l'algorithme de reconstruction mentionné plus haut. Cependant la lecture d'une ligne de la mémoire M n'est commandée que lorsque toutes les unités de mémorisation de cette ligne qui n'ont pas été «remplies» pendant la phase de mémorisation des vues $V_i$, auront reçu des informations numériques représentatives des valeurs intermédiaires, calculées par mise en œuvre de l'algorithme décrit ci-dessus. Pour cela, un processeur spécialisé ou un sous-programme de l'ordinateur effectue les opérations de calcul – d'adresse dans la mémoire M et d'interpolation linéaire avant de commander l'inscription des valeurs intermédiaires résultantes dans les unités de mémorisation laissées provisoirement «vides» lors de l'inscription des vues réelles dans cette même mémoire M.

Ce processeur ou ce sous-programme est symbolisé à la figure 1 par le bloc fonctionnel 22 agencé pour calculer les adresses des valeurs d'atténuation linéaires utiles, lire ces valeurs, effectuer le calcul d'interpolation combinant ces valeurs et réinscrire le résultat à l'adresse convenable d'une valeur intermédiaire d'une vue à compléter. Il pilote aussi les moyens de lecture 20 (liaison fonctionnelle 23) gérant le transfert des informations depuis la mémoire M vers le processeur de reconstruction 21, le transfert d'une «ligne» de la mémoire n'étant autorisé que lorsque toutes les unités de mémorisation correspondant aux valeurs intermédiaires ont été mémorisées.

## Revendications

1. Procédé de reconstruction d'image par tomodensitométrie, du type consistant à faire tourner un ensemble source-détecteurs dans le plan d'une coupe à imager, ladite source émettant un faisceau de rayonnement pénétrant en forme d'éventail et à relever et mémoriser une succession de vues pour différents angles $\beta$ de rotation de l'ensemble source-détecteurs par rapport à un système d'axes de référence fixe (XOY) dudit plan, lesdits détecteurs étant agencés pour converger vers le foyer (F) de ladite source et chaque vue étant échantillonnée pour des angles $\gamma$ représentatifs des détecteurs et repérés à partir de la droite (OF) joignant le centre de rotation (O) de l'ensemble source-détecteurs et ledit foyer (F), le décalage angulaire entre deux détecteurs adjacents étant $\Delta\gamma$ constant et le décalage angulaire $\Delta\beta$ entre vues étant constant, le décalage angulaire entre ladite droite (OF) et le détecteur le plus proche de celle-ci étant réglé à $\left[\dfrac{\Delta\gamma}{4}\right]$ :

– à relever et mémoriser une série de vues réparties sur un tour complet dudit ensemble source-détecteurs;

– à élaborer pour chaque vue ($V_1$) un nombre de valeurs ($s_j$) intermédiaires représentant un échantillonnage entrelacé avec celui desdits détecteurs ($d_i$), chaque valeur correspondant à un angle $\gamma$ d'échantillonnage donné étant obtenue par une combinaison d'au moins deux valeurs d'absorption linéaires,

– à appliquer un algorithme de reconstruction comprenant une convolution et une rétroprojection aux vues ainsi complétées par lesdites valeurs intermédiaires, caractérisé en ce que ces valeurs d'absorption linéaire sont déduites de valeurs de mesure prélevées dans des vues consécutives situées de part et d'autre (KG, KD) d'une vue fictive (VF) dont l'image (VF') par une transformation $T(\theta,\gamma) = (\theta + [2k-1]\pi, -\gamma)$ contient le point représentatif de ladite valeur intermédiaire dans l'espace $[\theta,\gamma]$ avec $\theta = \beta + \gamma$ et k entier relatif, lesdites valeurs de mesure desdites vues étant prélevées sur le détecteur d'angle ($\gamma$) opposé ($-\gamma$) à celui de ladite valeur intermédiaire recherchée.

2. Procédé de reconstruction selon la revendication 1, caractérisé en ce que ladite combinaison consiste, pour chaque valeur intermédiaire, à faire la moyenne entre les deux valeurs d'absorption linéaire précitées en les pondérant respectivement par l'inverse de l'écart, en projection sur l'axe $\vec{\theta}$ dans l'espace $[\theta, \gamma]$, entre leurs positions respectives résultant de ladite transformation T et la position de la valeur intermédiaire considérée.

## Patentansprüche

1. Verfahren zur Rekonstruktion eines Bildes durch Tomodensitometrie, welches darin besteht, eine Gruppe aus Quelle und Detektoren in der Ebene eines darzustellenden Schnittes rotieren zu lassen, wobei diese Quelle ein fächerförmig durchdringendes Strahlungsbündel ausstrahlt, sowie eine Folge von Ansichten für verschiedene Drehwinkel $\beta$ der Gruppe aus Quelle und Detektoren in bezug auf ein festes Referenz-Achsensystem (XOY) von dieser Ebene aufzunehmen und abzuspeichern, wobei diese Detektoren so angeordnet sind, daß sie zum Brennpunkt (F) dieser Quelle konvergieren, und jede Ansicht für Winkel $\gamma$ abgetastet wird, welche für die Detektoren repräsentativ sind und ausgehend von der Geraden (OF) markiert werden, die das Drehzentrum (O) der Gruppe aus Quelle und Detektoren mit diesem Brennpunkt (F) verbindet, wobei die Win-

kelversetzung $\Delta\gamma$ zwischen zwei benachbarten Detektoren konstant ist, die Winkelversetzung $\Delta\beta$ zwischen den Ansichten konstant ist und die Winkelversetzung zwischen dieser Geraden (OF) und dem ihr am nächsten stehenden Detektor eingestellt ist auf $\left\lfloor\dfrac{\Delta\gamma}{4}\right\rfloor$:

— eine Reihe von auf eine vollständige Drehung dieser Gruppe aus Quelle und Detektoren verteilten Ansichten aufzunehmen und abzuspeichern,

— für jede Ansicht ($V_1$) eine Anzahl von Zwischenwerten ($s_i$) zu erzeugen, welche eine mit derjenigen dieser Detektoren ($d_i$) verschachtelte Abtastung darstellt, wobei jeder einem bestimmten Abtastwinkel $\gamma$ entsprechende Wert durch eine Kombination von wenigstens zwei Werten einer linearen Absorption erhalten wird,

— einen Rekonstruktionsalgorithmus anzuwenden, welcher eine Faltung sowie eine Rückprojektion zu den durch diese Zwischenwerte so vervollständigten Ansichten umfaßt, dadurch gekennzeichnet, daß diese Werte einer linearen Absorption sich aus Meßwerten ergeben, welche in aufeinanderfolgenden, beiderseits (KG, KD) einer fiktiven Ansicht (VF) sich befindenden Ansichten abgenommen werden, wovon die Abbildung (VF') durch eine Transformation $T(\theta,\gamma) = (\theta + [2k-1]\pi, -\gamma)$ den Punkt enthält, der für diesen Zwischenwert im Raum $[\theta,\gamma]$ repräsentativ ist, worin $\theta = \beta + \gamma$ und $k$ eine ganze relative Zahl ist, wobei diese Meßwerte dieser Ansichten an dem Detektor für den Winkel $(\gamma)$, der demjenigen $(-\gamma)$ dieses gesuchten Zwischenwertes entgegengesetzt ist, abgenommen werden.

2. Verfahren zur Rekonstruktion nach Anspruch 1, dadurch gekennzeichnet, daß für jeden Zwischenwert diese Kombination daraus besteht, den Mittelwert zwischen zwei genannten Werten einer linearen Absorption zu errechnen, indem sie jeweils gewichtet werden mit dem Kehrwert der Differenz, bei Projektion auf die Achse $\overrightarrow{\theta}$ im Raume $[\theta, \gamma]$ zwischen ihren jeweiligen, aus der Transformation T resultierenden Position und dem betrachteten Zwischenwert.

## Claims

1. A tomodensitometric image reconstruction method of the type consisting in turning an assembly with a source and detectors in a plane of a section to be visualized, said source emitting a beam with a fan-like form of penetration, and in detecting and storing a succession of views for different angles $\beta$ of rotation of the source detector assembly in relation to a fixed reference axis system (XOY) of the said plane, said detectors being operated so as to converge on the focus (F) of the said source and each view being calibrated for the angles $\gamma$ representative of the detectors and labeled starting from the straight line (OF) connecting the center of rotation (O) of the source detector assembly and the said focus (F), the angular shift between two adjacent detectors being $\Delta\gamma$ and constant and the angular shift $\Delta\beta$ between views being constant, the angular shift between the said straight line (OF) and the detector nearest thereto being regulated to be $\left\lfloor\dfrac{\Delta\gamma}{4}\right\rfloor$:

— detecting and storing as series of views distributed over a full revolution of the said source detector assembly,

— elaborating for each view ($V_1$) a numer of intermediate values ($s_i$) representing a calibration interlaced with that of the said detectors ($d_i$), each value corresponding to an angle of a data calibration being obtained by a combination of at least two linear absorption values,

— and applying a reconstruction algorithm comprising one convolution and one retroprojection to the views thus completed with the said intermediate values, characterized in that these linear absorption values are deduced from the measurement values detected in consecutive situated on either side (KG and KD) of a fictive view (VF) whose image (VF') subject to a transformation $T(\theta,\gamma) = (\theta + [2k-1]\pi, -\gamma)$ contains the point representative of the said intermediate value in the space $[\theta,\gamma]$ with $\theta = \beta + \gamma$ and $k$ being a relative whole number, the said measurement values of the said views being detected using a detector for the angle $(\gamma)$ opposite $(-\gamma)$ to that of the said sought intermediate angle.

2. The reconstruction method as claimed in claim 1, characterized in that for each intermediate value, the said combination consists in deriving the average for the two said absorption values and weighting them respectively by the inverse of the offset, in a projection on the axis $\overrightarrow{\theta}$ in the space $[\theta, \gamma]$, between their respective positions resulting from the said transformation T and the positions of the intermediate value considered.

FIG_1

FIG_ 2

FIG_ 3

FiG_4

FiG_5

# FiG_6

# FiG_7

EP 0 159 248 B1

FIG_8